# EUROPÄISCHE PATENTANMELDUNG

(11) **EP 2 187 212 A1**
(43) Veröffentlichungstag der Anmeldung: **19.05.2010**
(21) Anmeldenummer: 09014279.5
(22) Anmeldetag: 16.11.2009
(51) Int. Cl.: G01N 33/542, G01N 33/68

(54) **Verfahren zur räumlich hochauflösenden Untersuchung einer mit einer Substanz markierten Struktur einer Probe**

(30) Priorität: 18.11.2008 DE 102008058088
(71) Anmelder: Leica Microsystems CMS GmbH, 35578 Wetzlar (DE)
(72) Erfinder: Sieber, Jochen, Dr., 68163 Mannheim (DE)
(74) Vertreter: Hassenbürger, Anneliese

(57) **Zusammenfassung**

Ein Verfahren zur räumlich hochauflösenden Untersuchung einer mit einer Substanz (3) markierten Struktur (1) einer Probe, wobei als Struktur (1), die mit der Substanz (3) markiert wird, eine biologische Struktur (1) verwendet wird, wobei die Substanz (3) von einem ersten Zustand in einen zweiten Zustand überführbar ist, wobei sich der erste und der zweite Zustand in mindestens einer fotophysikalischen Eigenschaft voneinander unterscheiden und wobei zur Markierung der Struktur (1) zunächst eine Bindung eines geeigneten Protein-Tags (2) an die Struktur (1) oder eine Expression des Tags (2) zusammen mit der Struktur (1) als Fusionsprotein erfolgt und das Tag (2) dann die Substanz (3) bindet, ist in vorteilhafter Weise derart weitergebildet, dass das Tag (2) ein FAP (Fluorogen Activating Protein) aufweist.

## Beschreibung

Die vorliegende Erfindung betrifft ein Verfahren zur räumlich hochauflösenden Untersuchung einer mit einer Substanz markierten Struktur einer Probe, wobei als Struktur, die mit der Substanz markiert wird, eine biologische Struktur verwendet wird, wobei die Substanz von einem ersten Zustand in einen zweiten Zustand überführbar ist, wobei sich der erste und der zweite Zustand in mindestens einer fotophysikalischen Eigenschaft voneinander unterscheiden und wobei zur Markierung der Struktur zunächst eine Bindung eines geeigneten Protein-Tags an die Struktur oder eine Expression des Tags zusammen mit der Struktur als Fusionsprotein erfolgt und das Tag dann die Substanz bindet.

Verfahren der eingangs genannten Art sind aus der Praxis bekannt, so bspw. aus der DE 10 2006 045 607 A1. Aus diesem Dokument ist es bspw. bekannt, zur Markierung des Proteins einen die fluoreszierende Substanz umfassenden Ligandkomplex über eine enzymatische Reaktion in der Zelle an ein Enzym zu binden. Dabei wird das Enzym als Fusionsprotein gemeinsam mit dem zu untersuchenden Protein exprimiert.

Als fotophysikalische Eigenschaft könnte bspw. die Eigenschaft betrachtet werden, Licht zu absorbieren oder vom angeregten in den Grundzustand zurückzukehren, oder es könnte der Querschnitt für den verbotenen Übergang in den Triplet-Zustand etc. betrachtet werden.

Bei dem bekannten Verfahren wird als Enzym bspw. ein genetisch modifiziertes Hydrolase-Protein verwendet, bei dem die katalytische Base gegen einen Phenylalanin-Rest ersetzt ist. An dieses Enzym kann ein Farbstoff über einen Linker ankoppeln.

Das bekannte Verfahren ist insoweit problematisch, als es letztendlich aufgrund technischer Limitationen (z. B. des Signals unspezifischer Hintergrundfärbung) ein Verfahren mit einem insgesamt begrenzten Anwendungsbereich ist. Es kann nicht allen Anforderungen unterschiedlicher Untersuchungstechniken genügen.

Der vorliegenden Erfindung liegt daher die Aufgabe zugrunde, ein Verfahren der eingangs genannten Art derart auszugestalten und weiterzubilden, dass bezüglich bekannter Anwendungsbereiche weitere alternative Anwendungsbereiche mit räumlich hochauflösender Untersuchungsqualität eröffnet sind.

Die voranstehende Aufgabe ist durch ein Verfahren mit den Merkmalen des Patentanspruchs 1 gelöst. Danach ist das bekannte Verfahren zur räumlich hochauflösenden Untersuchung einer mit einer Substanz markierten Struktur einer Probe derart ausgestaltet und weitergebildet, dass das Tag ein FAP (Fluorogen Activating Protein) aufweist.

In erfindungsgemäßer Weise ist zunächst erkannt worden, dass die voranstehende Aufgabe durch geschickte Auswahl des Tag auf überraschend einfache Weise gelöst wird. Hierzu ist in weiter erfindungsgemäßer Weise erkannt worden, dass die Verwendung eines FAP (Fluorogen Activating Protein) besondere Vorteile für unterschiedliche weitere Untersuchungsmethoden mit sich bringt. Dabei ist mit dem erfindungsgemäßen Verfahren insbesondere eine nahezu hintergrundfreie hochauflösende Untersuchung der markierten Struktur in einer lebenden Zelle möglich.

FAPs sind beispielsweise aus der WO 2004/025268 A2 oder aus der WO 2008/092041 A2 bekannt. Diese Proteine zeichnen sich durch ihre Wechselwirkung mit Fluorogenen wie beispielsweise Thiazolorange (TO) und Malachitgrün (MG) aus. Diese Wechselwirkung zur Erzeugung von Fluoreszenzeffekten ist ebenfalls in der Zeitschrift "nature biotechnology", Vol. 26, Nr. 2, Februar 2008, Seiten 235 bis 240 beschrieben.

Folglich ist mit dem erfindungsgemäßen Verfahren ein Verfahren angegeben, bei dem bezüglich bekannter Anwendungsbereiche alternative weitere Anwendungsbereiche mit räumlich hochauflösender Untersuchungsqualität eröffnet sind.

Im Konkreten könnte es sich bei dem ersten Zustand um einen nicht fluoreszierenden Zustand und bei dem zweiten Zustand um einen fluoreszierenden Zustand handeln. Die Interaktion der Substanz mit dem Tag bewirkt dann einen Übergang vom ersten in den zweiten Zustand.

Bei einer weiter vorteilhaften Ausgestaltung des Verfahrens könnte die Substanz nur im mit dem FAP gebundenen Zustand von dem ersten Zustand in den zweiten Zustand überführbar oder schaltbar sein. Insoweit können ganz spezifisch nur diejenigen Teile der Substanz in den zweiten Zustand überführt oder geschaltet werden, die auch wirklich mit dem zu markierenden Protein gekoppelt sind. Weitere in die Zelle eingebrachte Substanzen "stören" die Strukturdarstellung durch bspw. ein ungewünschtes Leuchten nicht.

Alternativ hierzu könnte die Substanz nur im mit dem FAP nicht gebundenen Zustand von dem ersten Zustand in den zweiten Zustand überführbar oder schaltbar sein.

Je nach Anwendungsfall könnte die Bindung des Tags an die Struktur kovalent sein. Alternativ hierzu könnte die Bindung nicht kovalent sein, wobei auch hier auf den jeweiligen Anwendungsfall abzustellen ist. In vorteilhafter Weise könnte die nicht kovalente Natur der Bindung zwischen der Substanz und dem Tag dazu führen, dass eine Ablösung eines bereits an das Tag angekoppelten Substanzbestandteils von dem Tag und das Eingehen einer neuen Bindung desselben Substanzbestandteils an einem anderen Tag und/oder eine neue Bindung an dem ursprünglichen Tag mit einem neuen Substanzbestandteil ermöglicht sind.

In weiter vorteilhafter Weise könnte die Bindung eine an die jeweilige Applikation angepasste Affinität aufweisen. Häufig ist Bleichen ein Problem bei hochauflösenden Untersuchungsverfahren. Langzeitbeobachtungen sind dabei meist nicht möglich, da ein bereits gebleichter Substanzbestandteil nicht mehr zur Erzeugung von Fluoreszenz genutzt werden kann. In vorteilhafter Weise könnte daher ein während des an das FAP gebundenen Zustands geblichenes Molekül der Substanz durch ein nicht geblichenes Molekül der Substanz ersetzt werden. In vorteilhafter Weise könnte die Affinität derart angepasst sein, dass eine jeweils optimale Austauschrate der Substanz am FAP ermöglicht ist. Hierbei könnte eine verhältnismäßig niedrige Affinität von großem Vorteil sein, da diese einen schnelleren Austausch von geblichenen mit nicht geblichenen Molekülen ermöglicht. Damit sind auch Langzeitaufnahmen auch mit stärker bleichenden Methoden ermöglicht. Die Problematik des Bleichens ist damit erheblich reduziert. In der Ausführungsform, in der die Substanz nur in der an das Tag gebundenen Form fluoreszieren würde, wäre eine hohe Hintergrundkonzentration in der Probe nicht von Belang, da von dieser kein Signal ausgehen würde.
Im Konkreten könnte das Tag einen die Struktur bindenden Teil und einen die Substanz bindenden Teil aufweisen. Beide Teile könnten für ihre jeweilige Aufgabe spezialisiert sein.

In weiter vorteilhafter Weise könnte der die Struktur bindende Teil nicht kovalent an den die Substanz bindenden Teil binden.

Je nach Anwendungsfall und Erfordernis könnte zwischen dem die Struktur bindenden Teil und dem die Substanz bindenden Teil mindestens ein Adapterprotein realisiert werden. Die Anzahl der verwendeten Adapterproteine ist je nach Anwendungsfall zu wählen.

In weiter vorteilhafter Weise könnte eine oder könnten mehrere fotophysikalische Eigenschaften der Substanz durch die Bereitstellung oder Veränderung von vorzugsweise lokalen Umgebungsbedingungen, z. B. die Bindung oder die Art der Bindung an das FAP, beeinflussbar sein. Dabei könnte es sich um eine Eigenschaft handeln, die bei der Hochauflösung ausgenutzt werden kann. So können genau die gewünschten Eigenschaften der Substanz erreicht werden, die für die hochauflösende Untersuchungsmethode bedeutsam sind. Hierdurch können die erhaltenen Ergebnisse stark verbessert werden. Insbesondere könnte die Überführbarkeit der Substanz vom einen in den anderen Zustand beeinflussbar sein.

Bei einer weiter konkreten Ausgestaltung des Verfahrens könnte die Substanz in Abhängigkeit von der verwendeten Untersuchungstechnik oder einer Eigenschaft der verwendeten Untersuchungstechnik ausgewählt oder angepasst werden. Nicht nur der Substanz-Typ an sich könnte ausgewählt oder angepasst werden. Auch eine Auswahl oder Anpassung einer Kombination unterschiedlicher Substanzen ist hier denkbar, um die Untersuchungsergebnisse zu verbessern.

Als Eigenschaften der Substanz, die für eine jeweilige Untersuchung bedeutsam sind, können die Wechselwilligkeit hinsichtlich eines Übergangs in den Triplet-Zustand, eines Dunkel-Zustands oder das Vorliegen eines großen Querschnitts für stimulierte Emission oder die Fähigkeit, fotoaktivierbar zu sein, berücksichtigt werden.

Des Weiteren könnte alternativ oder zusätzlich hierzu der FAP-Typ in Abhängigkeit von der verwendeten Untersuchungstechnik oder einer Eigenschaft der verwendeten Untersuchungstechnik ausgewählt oder angepasst werden.

In besonders vorteilhafter Weise könnten sowohl die FAPs als auch die jeweilige fluoreszierende Substanz auf die für die jeweilige hochauflösende Untersuchungsmethode wichtige Eigenschaft optimiert werden, um so bessere Ergebnisse zu erzielen. Dabei besteht die Möglichkeit, die Markierung auf die verwendete Untersuchungstechnik zu optimieren. Insbesondere auf der FAP-Seite ist dies durch die heutige Molekularbiologie und die verfügbaren Screening-Methoden leicht zu erreichen. Das FAP kann so verändert werden, dass das Bindeverhalten, die Affinität und evtl. auch die fotophysikalischen Eigenschaften der verwendeten Substanz auf den jeweiligen Anwendungsfall hin optimiert werden.

Durch die Verwendung verschiedener Substanzen mit einem gegebenen FAP oder einer gegebenen Substanz mit verschiedenen FAPs, kann leicht die geeignete Kombination für den jeweiligen Anwendungsfall erreicht werden.

In besonders vorteilhafter Weise könnte das Verfahren im Bereich der STED-Mikroskopie eingesetzt werden. Alternativ oder zusätzliche hierzu könnte das Verfahren im Bereich der RESOLFT-, GSD, DSO- oder SSI-Mikroskopie eingesetzt werden. Letztendlich ist mit dem erfindungsgemäßen Verfahren eine FAP-Nanoskopie bei lebenden Zellen möglich.

Durch eine geeignete Kombination einer fluoreszierenden Substanz mit einem FAP eröffnet sich die Möglichkeit, Strukturen mit Fluorophoren oder Fluoreszenzfarbstoffen auch in lebenden Zellen zu markieren, die die für die entsprechende Technik geeigneten Eigenschaften aufweisen, bspw. eine hohe oder niedrige Tendenz, in den Triplet-Zustand zu gelangen, ein geringes Bleichen, einen großen Querschnitt für stimulierte Emission usw..

Ein Vorteil der FAP-Technologie ist, dass durch die Bindung der Substanz, Fluorophor bzw. Fluorogen, an das FAP die Eigenschaften der Substanz, Fluorophor bzw. Fluorogen, verändert werden können. Bspw. könnte diese zunächst nicht fluoreszieren oder leuchten, sondern erst nach Eingehen einer Bindung mit dem FAP.
Hinsichtlich der Reduktion des Problems des Bleichens ist von Vorteil, dass die Substanz-FAP-Bindung nicht kovalent ist. Damit kann sich die Substanz, Fluorophor bzw. Fluorogen, von der Bindungsstelle lösen und eine neue Bindung eingehen. Sollte die Substanz zuvor geblichen worden sein, kann sie so durch eine nicht geblichene Substanz ersetzt werden. Dies ist ein klarer Vorteil, der Langzeitaufnahmen auch mit stärker bleichenden Methoden ermöglicht. Es ist daher unter Umständen sinnvoll, Substanz-FAP-Kombinationen von vergleichsweise niedriger Affinität zu verwenden, um so die Problematik des Bleichens zu reduzieren.

Letztendlich ist bei der beanspruchten Lehre von Bedeutung, dass Substanz-FAP-Kombinationen für die Markierung von Strukturen für die Superresolution-Mikroskopie verwendet werden, in der bestimmte Eigenschaften der Substanz zum Erreichen der Höchstauflösung verwendet werden. Des Weiteren ist die gezielte Optimierung bestimmter Eigenschaften der Substanz von Bedeutung, die für die entsprechenden Superresolution-Techniken ausschlaggebend sind, wobei die Bindung an ein entsprechend ausgewähltes FAP beeinflusst werden kann.

Es gibt nun verschiedene Möglichkeiten, die Lehre der vorliegenden Erfindung in vorteilhafter Weise auszugestalten und weiterzubilden. Dazu ist einerseits auf die dem Patentanspruch 1 nachgeordneten Ansprüche und andererseits auf die nachfolgende Erläuterung bevorzugter Ausführungsbeispiele der Erfindung anhand der Zeichnung zu verweisen. In Verbindung mit der Erläuterung der bevorzugten Ausführungsbeispiele der Erfindung anhand der Zeichnung werden auch im Allgemeinen bevorzugte Ausgestaltungen und Weiterbildungen der Lehre erläutert. In der Zeichnung zeigen
- Fig. 1: in einer schematischen Darstellung ein Ausführungsbeispiel eines Bindungsprozesses zwischen Substanz und Tag, wobei im oberen Bereich der Fig. 1 eine kovalente Bindung und im unteren Bereich der Fig. 1 eine nicht kovalente Bindung zwischen Tag und Struktur vorliegt, und
- Fig.2: in einer schematischen Darstellung ein weiteres Ausführungsbeispiel eines Bindungsprozesses zwischen Substanz und Tag, wobei im oberen Bereich der Fig. 2 wiederum eine kovalente Bindung und im unteren Bereich der Fig. 2 eine nicht kovalente Bindung zwischen Tag und Struktur dargestellt ist.

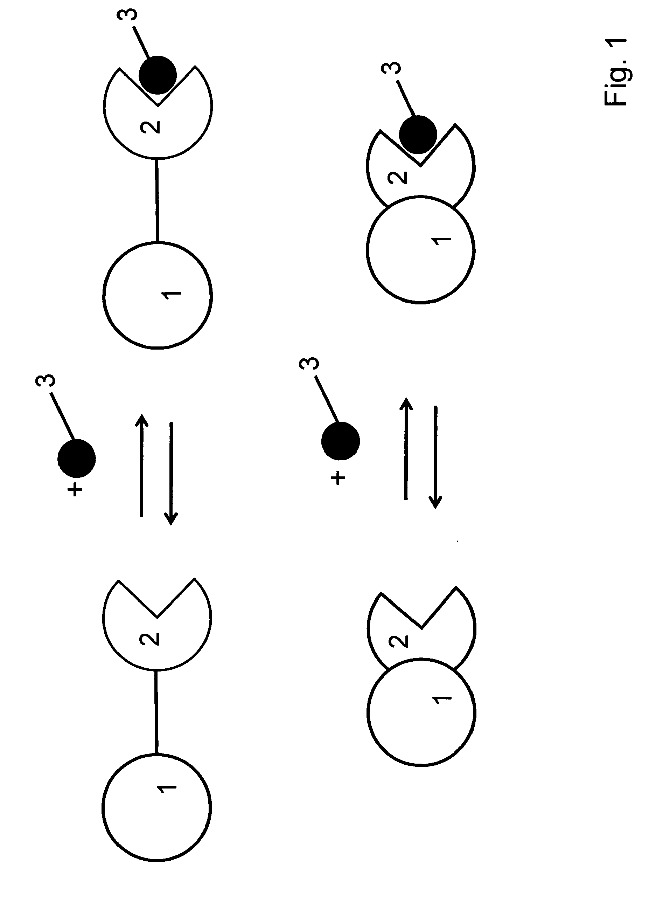

Fig. 1 zeigt in einer schematischen Darstellung zwei verschiedene Bindungstypen zwischen einer Struktur 1 und einem Tag 2. Im oberen Bereich der Fig. 1 ist eine kovalente Bindung zwischen der Struktur 1 und dem Tag 2 realisiert. Im unteren Bereich der Fig. 1 ist eine nicht kovalente Anbindung des Tags 2 an die Struktur 1 gezeigt.

Des Weiteren ist in Fig. 1 die Hinzufügung einer markierenden Substanz 3 an das Tag 2 gezeigt. Der Doppelpfeil zwischen den Zuständen ohne angebundene Substanz 3 und mit angebundener Substanz 3 deutet darauf hin, dass sich die Substanz 3 nach ihrer Bindung an das Tag 2 auch wieder von dem Tag 2 lösen kann.

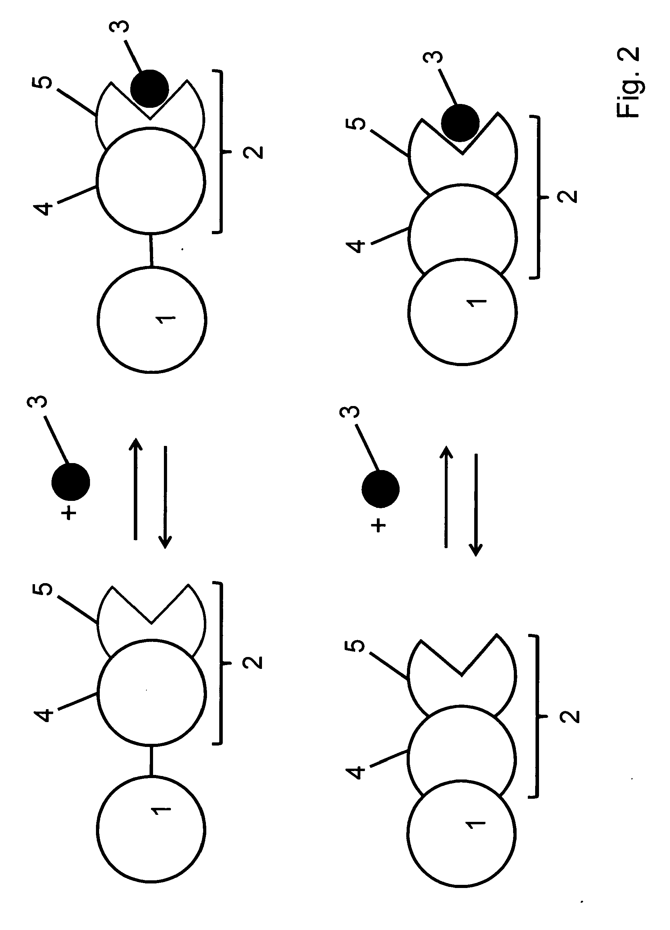

Fig. 2 ist ähnlich aufgebaut wie Fig. 1, wobei im oberen Bereich eine kovalente Anbindung der Struktur 1 an das Tag 2 und im unteren Bereich eine nicht kovalente Anbindung der Struktur 1 an das Tag 2 gezeigt ist. In beiden Bereichen ist ebenfalls eine Hinzufügung einer markierenden Substanz 3 an das Tag 2 gezeigt.

Im Unterschied zur Fig. 1 besteht das Tag 2 gemäß dem in Fig. 2 gezeigten Ausführungsbeispiel aus einem die Struktur 1 bindenden Teil 4 und aus einem die Substanz 3 bindenden Teil 5.

Der Teil 4 ist nicht kovalent an den Teil 5 angebunden. Zwischen dem die Struktur 1 bindenden Teil 4 und dem die Substanz 3 bindenden Teil 5 könnte mindestens ein Adapterprotein angebunden sein.

Abschließend sei hervorgehoben, dass die voranstehend beschriebenen Ausführungsbeispiele lediglich zur Erörterung der beanspruchten Lehre dienen, diese jedoch nicht auf die Ausführungsbeispiele einschränken.

### Bezugszeichenliste

- 1: Struktur
- 2: Tag
- 3: Substanz
- 4: Teil
- 5: Teil

## Patentansprüche

1. Verfahren zur räumlich hochauflösenden Untersuchung einer mit einer Substanz (3) markierten Struktur (1) einer Probe, wobei als Struktur (1), die mit der Substanz (3) markiert wird, eine biologische Struktur (1) verwendet wird, wobei die Substanz (3) von einem ersten Zustand in einen zweiten Zustand überführbar ist, wobei sich der erste und der zweite Zustand in mindestens einer fotophysikalischen Eigenschaft voneinander unterscheiden und wobei zur Markierung der Struktur (1) zunächst eine Bindung eines geeigneten Protein-Tags (2) an die Struktur (1) oder eine Expression des Tags (2) zusammen mit der Struktur (1) als Fusionsprotein erfolgt und das Tag (2) dann die Substanz (3) bindet,
**dadurch gekennzeichnet, dass** das Tag (2) ein FAP (Fluorogen Activating Protein) aufweist.

2. Verfahren nach Anspruch 1, **dadurch gekennzeichnet, dass** es sich bei dem ersten Zustand um einen nicht fluoreszierenden Zustand und bei dem zweiten Zustand um einen fluoreszierenden Zustand handelt.

3. Verfahren nach Anspruch 1 oder 2, **dadurch gekennzeichnet, dass** die Substanz (3) nur im mit dem FAP gebundenen Zustand von dem ersten Zustand in den zweiten Zustand überführbar oder schaltbar ist.

4. Verfahren nach Anspruch 1 oder 2, **dadurch gekennzeichnet, dass** die Substanz (3) nur im mit dem FAP nicht gebundenen Zustand von dem ersten Zustand in den zweiten Zustand überführbar ist.

5. Verfahren nach einem der Ansprüche 1 bis 4, **dadurch gekennzeichnet, dass** die Bindung kovalent ist.

6. Verfahren nach einem der Ansprüche 1 bis 4, **dadurch gekennzeichnet, dass** die Bindung nicht kovalent ist.

7. Verfahren nach einem der Ansprüche 1 bis 6, **dadurch gekennzeichnet, dass** ein während des an das FAP gebundenen Zustands geblichenes Molekül der Substanz (3) durch ein nicht geblichenes Molekül der Substanz (3) ersetzt wird.

8. Verfahren nach einem der Ansprüche 1 bis 7, **dadurch gekennzeichnet, dass** das Tag (2) einen die Struktur (1) bindenden Teil (4) und einen die Substanz (3) bindenden Teil (5) aufweist.

9. Verfahren nach Anspruch 8, **dadurch gekennzeichnet, dass** der die Struktur (1) bindende Teil (4) nicht kovalent an den die Substanz (3) bindenden Teil (5) bindet.

10. Verfahren nach Anspruch 8 oder 9, **dadurch gekennzeichnet, dass** zwischen dem die Struktur (1) bindenden Teil (4) und dem die Substanz (3) bindenden Teil (5) mindestens ein Adapterprotein realisiert wird.

11. Verfahren nach einem der Ansprüche 1 bis 10, **dadurch gekennzeichnet, dass** eine oder mehrere fotophysikalische Eigenschaften der Substanz (3) durch die Bereitstellung oder Veränderung von vorzugsweise lokalen Umgebungsbedingungen beeinflussbar ist.

12. Verfahren nach Anspruch 11, **dadurch gekennzeichnet, dass** die Veränderung der fotophysikalischen Eigenschaft oder Eigenschaften durch die Bindung oder die Art der Bindung an das FAP erfolgt.

13. Verfahren nach einem der Ansprüche 1 bis 12, **dadurch gekennzeichnet, dass** der FAP-Typ in Abhängigkeit von der verwendeten Untersuchungstechnik oder einer Eigenschaft der verwendeten Untersuchungstechnik ausgewählt oder angepasst wird.

14. Verfahren nach einem der Ansprüche 1 bis 13, **dadurch gekennzeichnet, dass** das Verfahren im Bereich der STED-Mikroskopie eingesetzt wird.

15. Verfahren nach einem der Ansprüche 1 bis 14, **dadurch gekennzeichnet, dass** das Verfahren im Bereich der RESOLFT-, GSD-, DSO- oder SSI-Mikroskopie eingesetzt wird.
